# EUROPÄISCHE PATENTSCHRIFT

(11) **EP 1 216 693 B1**
(45) Veröffentlichungstag und Bekanntmachung des Hinweises auf die Patenterteilung: **08.08.2007**
(21) Anmeldenummer: 01129179.6
(22) Anmeldetag: 10.12.2001
(51) Int. Cl.: A61K 8/81, A61Q 1/02, A61Q 15/00, A61Q 17/00, A61Q 19/00

(54) **Lidschatten auf Gelbasis mit einem Gehalt an einem oder mehreren Ammoniumacryloyldimethyltaurat/Vinylpyrrolidoncopolymeren**
Eye shadow composition containing at least one ammoniumacryloyldimethyltaurate/vinylpyrrolidone copolymer
Ombre pour paupières contenant au moins un copolymère d'ammoniumacryloyldimethyltaurate/vinylpyrrolidone

(30) Priorität: 23.12.2000 DE 10065044
(43) Veröffentlichungstag der Anmeldung: 26.06.2002
(73) Patentinhaber: CLARIANT INTERNATIONAL LTD., 4132 Muttenz (CH)
(72) Erfinder: Lanzendörfer, Ghita, Dr., 22087 Hamburg (DE); Bormann, Angelika, 22041 Hamburg (DE)

(56) Entgegenhaltungen:
- EP-A- 0 522 756
- EP-A- 0 815 828
- EP-A- 1 035 181
- US-A- 5 034 216

## Beschreibung

Die vorliegende Erfindung betrifft Lidschatten auf Gelbasis mit einem Gehalt an einem oder mehreren Ammoniumacryloyldimethyltaura/Vinylpyrrolidoncopolymeren, Verfahren zu ihrer Herstellung sowie ihre Verwendung.

Von den Augen geht eine Signalwirkung vieler Emotionen aus. Die Augen sind der Teil des Gesichts, der in der Mimik die größte Bedeutung hat. Es ist daher folgerichtig, der Augenpartie besonderes kosmetisches Gewicht zu verleihen. Dies wird seit Jahrtausenden durch unterschiedliche Zubereitungen getan, beispielsweise durch Lidschatten.

Wegen der Empfindlichkeit des Auges muß gewährleistet sein, daß zwar einesteils die gewünschte dekorative Wirkung erzielt wird, daß die Zubereitungen selbst aber andererseits keine Reizwirkung entfalten.

Übliche, und sich gerade in neuerer Zeit immer weiter verbreitende kosmetische und dermatologische Zubereitungsformen sind Gele.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrückenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind. Meistens sind Gele durchsichtig. In der kosmetischen bzw. pharmazeutischen Galenik zeichnen sich Gele in aller Regel durch halbfeste, oft fließfähige Konsistenz aus.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten. Klare Gele sind für die optimale Farbentwicklung der Formel ideal. Nachteilig wirkt sich aber häufig aus, daß zum Erhalt einer stabilen Formulierung relativ viel Gelbildner verwendet werden muß und eine klebrige unästhetische Sensorik erreicht wird.

Diesen Übelständen galt es, Abhilfe zu schaffen.

Erstaunlicherweise werden diese Aufgaben gelöst durch Lidschatten auf Gelbasis mit einem Gehalt an einem oder mehreren Ammoniumacryloyldimethyltaurat/Vinylpyrrolidoncopolymeren, ferner enthaltend einen oder mehrere Farbstoffe und/oder Farbpigmente, wobei die Gesamtmenge der Farbstoffe und farbgebenden Pigmente aus dem Bereich von 0,1 Gew.-% bis 30 Gew.-%, vorzugsweise von 0,5 bis 15 Gew.-%, insbesondere von 1,0 bis 10 Gew.-% ausgewählt wird, jeweils bezogen auf das Gesamtgewicht der Zubereitungen.

Gegenstand der vorliegenden Erfindung ist daher eine Lidschattenzubereitung, dadurch gekennzeichnet, dass sie auf einem kosmetischen Gel basiert und einen Gehalt an einem oder mehreren Ammoniumacryloyldimethyltaurat/Vinylpyrrolidoncopolymeren, sowie einen Gehalt an einem oder mehreren Farbstoffen und/oder Farbpigmenten aufweist und die Gesamtmenge der Farbstoffe und farbgebenden Pigmente aus dem Bereich von 0,1 Gew.-% bis 30 Gew,-%, vorzugsweise von 0.5 bis 15 Gew.-%, insbesondere von 1,0 bis 10 Gew.-% gewählt wird, jeweils bezogen auf das Gesamtgewicht der Zubereitungen.

In EP 0 522 756 werden Ampholytterpolymere als konditionierende Additive für Haut- und Nagelpflegeprodukte beschrieben. 2-Acrylamido-2-methylpropansulfonsäure und Vinylpyrrolidon werden u.a. als Monomere zur Herstellung der Terpolymere genannt.

In EP 0 815 828 werden vernetzte 2-Acrylamido-2-methylpropansulfonsäure-Polymere offenbart.

US 5,034.216 beschreibt wasserfreie, dreiphasige, pulverförmige kosmetische Produkte, die 10 bis 60 Gew.-% einer Gelphase enthalten. Die Gelphase enthält Ethylen/Acrylat-Copolymere und einen Ester der Formel ROCOR₁, worin R von einem Alkohol oder Fettalkohol mit einem Molekulargewicht von 60 - 400 und R₁ von einer Fettsäure mit einem Molekulargewicht von 120 - 500 abstammt.

In EP 1 035 181 wird eine pigmenthaltige Gelmasse auf Ölbasis beschrieben, die 0,1 bis 25 Gew.-% einer Hydroxyfettsäure mit 10 bis 20 C-Atomen, 0,1 bis 30 Gel.-% C₃₀₋₄₅-Alkylmethlcone, 1 bis 70 Gew.-% einer Ölkomponente und 0,1 bis 50 Gew.-% Pigmente enthält.

Erfindungsgemäß vorteilhaft weisen das oder die Ammoniumacryloyldimethyltaurate/Vinylpyrrolidoncopolymere die Summenformel [C₇H₁₆N₂SO₄]ₙ [C₈H₉NO]ₘ auf, einer statistischen Struktur wie folgt entsprechend

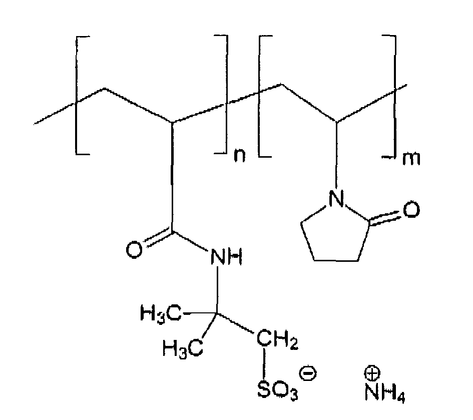

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Es war für den Fachmann nicht vorauszusehen gewesen, daß die erfindungsgemäßen Lidschatten
- kräftigere Farbschattierungen, auch bei geringerem Auftrag, erlauben,
- bessere sensorische Eigenschaften, wie beispielsweise die Verteilbarkeit auf der Haut oder das Einzugsvermögen in die Haut, aufweisen,
- höhere Stabilität aufweisen und
- sich durch bessere Bioverträglichkeit auszeichnen würden
als die Zubereitungen des Standes der Technik.

Die erfindungsgemäßen Zubereitungen stellen daher eine Bereicherung des Standes der Technik dar.

Es ist möglich und vorteilhaft, das oder die Ammoniumacryloyldimethyltaurate/Vinylpyrrolidoncopolymere zu jedem beliebigen Zeitpunkte der Gelherstellung zuzugeben.

Die erfindungsgemäßen kosmetischen Zubereitungen können ferner vorteilhaft weitere kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die erfindungsgemäßen Gele UVB-Filtersubstanzen, können diese vorteilhaft wasserlöslich sein. Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxy-benzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4' methoxyphenyl)propan-1,3-dion und um 1-Phenyl-3-(4'-isopropylphenyl)propan-1,3-dion. Es können die für die UVB-Kombination verwendeten Mengen eingesetzt werden.

Die wäßrige Phase der erfindungsgemäßen Gele enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe der Hydrocolloide.

Erfindungsgemäße als Gele vorliegenden Zubereitungen enthalten gegebenenfalls vor teilhaft ein oder mehrere zusätzliche Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Goodrich (Carbopol 980, 981, 1382, 5984, 2984, EDT 2001 oder Pemulen TR2).

Erfindungsgemäße Lidschattenzubereitungen können vorteilhaft, wenngleich nicht zwingend weitere Polymere enthalten, die zum Beispiel vorteilhaft gewählt werden aus der Gruppe der anionischen Polymere, der amphoteren oder zwitterionischen Polymere und der nichtionischen Polymere.

### Geeignete anionische Polymere sind beispielsweise:

Copolymere der Maleinsäure, Fumarsäure und Itaconsäure sowie der Anhydride und Halbester dieser Säuren mit Vinylethern, Vinylestern, Vinylhalogeniden, Phenylvinylderivaten und Acrylsäure und deren Estern, die u.a. von der Firma ISP unter den Handelsnamen GANTREZ® A, SP und ES bzw. OMNIREZ® 2000 angeboten werden.

Besonders bevorzugt wird hierbei die Type, die gemäß der INCI-Nomenklatur "Butyl Ester of PVM/MA Copolymer" genannt wird, wie sie beispielsweise unter den Handelsbezeichnungen GANTREZ® Super A-425 erhältlich ist.

Copolymere der Crotonensäure mit Vinylacetat oder Vinylpropionat und Crotonensäure/Vinylacetat/Vinylneodecanoaten-Terpolymeren, die gemäß der INCI-Nomenklatur "VA / Crotonates / Vinyl Neodecanoate Copolymer" genannt werden, und die unter den Handelsbezeichnungen RESYN® von der Firma National Starch bzw. LUVISET® von der Firma BASF verfügbar sind. Besonders bevorzugt wird hierbei die Type RESYN® 28-2942.

Homo- und Copolymere der Acryl- und Methacrylsäure und der Salze dieser Säuren, die mit den Handelsnamen RETEN® von der Firma Hercules und VERSICOL® von der Firma Allied Colloid angeboten werden.

Co- und Terpolymere der Acryl- oder Methacrylsäure mit Ethylen, Styrol, Vinyl- oder Allylestern, wie z.B. Vinylacetat, Vinylpyrrolidon oder Vinylcaprolactam, Estern der Acryl- und Methacrylsäure mit einem oder mehreren gesättigten Alkoholen, die auch auf ein Polyalkylenglykol gepfropft und vernetzt sein können, Acrylamid, Methacrylamid, N-Alkyl- und N-Hydroxyalkylsubstituierten Acryl- und Methacrylamiden.

Diese werden u.a. von der Firma BASF unter den Handelsnamen ULTRAHOLD®, LUVI-MER® und LUVIFLEX®, von der Firma National Starch mit den Bezeichnungen AM-PHOMER®, LOVOCRYL®, VERSATYL® und BALANCE®, der Firma ISP unter den Namen ACRYLIDONEO, ACCUDYNE® bzw. ADVANTAGE PLUS® und der Firma American Cyanamid mit dem Handelsnamen QUADRAMER® vertrieben.

Besonders bevorzugt werden hierbei die Typen ULTRAHOLD® 8 und Strong (INCI: Acrylates / Acrylamide Copolymer), LUVIMER® 100P (INCI: Acrylates Copolymer), BALAN-CE® 0/55 (INCI: Acrylates Copolymer) und ACCUDYNE ® 258 (INCI: Acrylates / Hydroxyesteracrylates Copolymer).

Polymere, die Vinylsulfonsäure-, Styrolsulfonsäure-, Naphthalinsulfonsäure- oder Acrylamidoalkylsulfonsäureeinheiten besitzen, wie z.B. Salze der Polyacrylamidsulfonsäuren und Copolymeren aus Acryl- oder Methacrylsäure und den Esten dieser Säuren und Acrylamid, Acrylamidderivaten, Vinylethern und Vinylpyrrolidon, oder Salze der Polystyrolsulfonsäure, die z.B. als Natriumsalze unter dem Handelsnamen FLEXAN® von der Firma National Starch angeboten werden.

### Geeignete amphotere Polymer sind:

Copolymere aus N-substituierten Alkylacryl- oder Methacrylamiden, wie z.B. N-Ethylacrylamid, N-tert.-butylacrylamid, N-octylacrylamid, Säuren, wie z.B. Acry-, Methacryl-, Crotonen-, Itacon-, Malein und Fumarsäure und der Alkylester dieser Säuren und basischen Einheiten bestehend aus Estern der Acryl- oder Methacrylsäure mit primären, sekundären und tertiären Aminsubstituenten oder Ammoniumsubstituenten, wie z.B. N-tert.-butylaminoethylmethacrylat.

Diese werden unter den Handelsbezeichnungen AMPHOMER®, AMPHOMER® LV-71 und BALANCE®-47 von der Firma National Starch angeboten, wobei der Typ AMPHO-MER® LV-71 (INCI: Octylacrylamide/Acrylates/Butylaminoethyl Methacrylate Copolymer) besonders bevorzugt wird.

Copolymere aus betainartigen Dialkylaminoalkyl(meth)acrylaten oder Dialkylaminoethyl-(meth)acrylamiden, die von den Firmen Mitsubishi Chemical Coop bzw. Clariant unter den Handelsnamen DIAFORMER® und YUKAFORMER® angeboten werden.

Zur Verbesserung der Wasserlöslichkeit bzw. Wasserdispergierbarkeit der anionischen und amphoteren Polymere können diese mit geeigneten Basen neutralisiert werden. Hierzu können Alkali- und Erdalkalihydroxide, Ammoniak und organische Amine, speziell Aminoalkohole, wie z.B. Triethanolamin, Triisopropanolamin, 2-Amino-2-methyl-1-propanol, 2-Amino-2-methyl-1,3-propandiol alleine oder in Mischungen eingesetzt werden. Besonders bevorzugt werden hierbei Natriumhydroxid und 2-Amino-2-methyl-1-propanol.

Die Neutralisation der freien Säuregruppen kann dabei je nach Anwendungszweck teilweise oder vollständig erfolgen, wobei ein Neutralisationsgrad von 80 - 100% bevorzugt wird.

Geeignete nichtionische Polymere sind beispielsweise
- Homopolymere des N-Vinylpyrrolidons, die als LUVISKOL® K-Typen von der Gesellschaft BASF bzw. PVP-K®-Typen von der Gesellschaft ISP mit verschiedenen mittleren Molmassen als Pulver oder in wässrigen bzw. wässrig/alkoholischen Lösungen angeboten werden. Bevorzugt wird hierbei der Typ Luviskol K30.
- Homopolymere des N-Vinylcaprolactam, z.B. von der Gesellschaft BASF unter den Handelsnamen LUVISKOL® Plus.
- Homopolymere des N-Vinylformamids.
- Copolymerisate aus N-Vinylpyrrolidon und Vinylacetat und/oder Vinylpropionat, die als LUVISKOL® VA-Typen von der Gesellschaft BASF bzw. PVP/VA ®-Typen von der Gesellschaft ISP in verschiedenen Konzentrationsverhältnissen als Pulver oder in wässrigen bzw. wässrig/alkoholischen Lösungen angeboten werden. Bevorzugt werden hier die Typen LUVISKOL® VA 37E und VA 64W
- Terpolymere aus N-Vinylpyrrolidon, Vinylacetat und Vinylpropionat, z.B. von der Gesellschaft BASF unter den Handelsnamen LUVISKOL® VAP 343. Besonders bevorzugt werden hierbei die nichtionischen Polymere LUVISKOL® Plus und LUVISKOL® VA 64W.

Die erfindungsgemäßen Lidschatten enthalten einen oder mehrere Farbstoffe und/oder Farbpigmente. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy- | 12490 | rot |
| 5"-chloro-2",4"-dimethoxy-2-naphthoesäureanilid | | |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3- | 15865 | rot |
| carbonsäure | | |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8- | 28440 | schwarz |
| acetyl-aminonaphthalin-3,5-disulfosäure | | |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophe- | 42053 | grün |
| nyl)-(methylen)-1-(N-ethylN-p-sulfobenzyl)-2,5-cyclohexadienimin] Acid Blue 7 | 42080 | blau |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein Chinophthalon | 45430 47000 | rot gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und | 75810 | grün |
| Chlorophylline | | |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂ · 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   - "Fischsilber" (Guanin/Hypoxanthin-Mischkristalle aus Fischschuppen) und
   - "Perlmutt" (vermahlene Muschelschalen)
2. Monokristalline Perlglanzpigmente wie z. B. Bismuthoxychlorid (BiOCI)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung / Schichtdicke** | **Farbe** |
|---|---|---|
| **Silberweiße Perlglanzpigmente** | TiO₂: 40 - 60 nm | silber |
| **Interferenzpigmente** | TiO₂: 60 - 80 nm | gelb |
| | TiO₂: 80 - 100 nm | rot |
| | TiO₂: 100 - 140 nm | blau |
| | TiO₂: 120 - 160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von SiO₂ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 µm zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraesslgsäure und deren Salze und andere mehr. Als Kationen der Salze werden bevorzugt Ammonium,- Alkylammonium,-Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Die erfindungsgemäßen Gelcrèmes können als Grundlage für kosmetische oder dermatologische Formulierungen dienen. Diese können wie üblich zusammengesetzt sein und beispielsweise zur Behandlung und der Pflege der Haut und als Schminkprodukt in der dekorativen Kosmetik dienen. Zur Anwendung werden die erfindungsgemäßen kosmetischen und dermatologischen Zubereitungen in der für Kosmetika oder Dermatika üblichen Weise auf die Haut in ausreichender Menge aufgebracht.

Die dünnflüssigen kosmetischen oder dermatologischen Mittel gemäß der Erfindung können beispielsweise als aus Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäurelsopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Derivate des 1,3,5-Triazins, vorzugsweise 2,4,6-Trianilino-(p-carbo-2'-ethyl-1'-hexyloxy) -1,3,5-triazin.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtem zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- odermonobutylether, Diethylenglykolmonomethyl- oder -monoethylether und analoge Produkte.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiel 1:

| | Gew.-% |
|---|---|
| PEG-8 (Polyethylenglycol 400) | 2,00 |
| Ethanol | 5,00 |
| Aristoflex AVC | 1,50 |
| Glycerin | 2,00 |
| Panthenol | 0,50 |
| Tocopherolacetat | 0,50 |
| Timiron Splendid blue ® (Merck KgaA) | 4,50 |
| Chromoxid grün | 1,00 |
| Parfüm, Konservierungsmittel, NaOH, Komplexbildner, | |
| Farbstoffe, Antioxidantien etc. | q.s. |
| Wasser | ad 100,00 |

### Beispiel 2:

| | Gew. % |
|---|---|
| Aristoflex AVC | 1,50 |
| Glycerin | 2,50 |
| 1,3 Butylenglycol | 2,50 |
| Perlglanzpigmente | 5,00 |
| EDTA | 0,20 |
| Parfüm, Konservierungsmittel, NaOH, | |
| Farbstoffe, Antioxidantien, etc. | q.s. |
| Wasser | ad 100,00 |

### Beispiel 3:

| | Gew. % |
|---|---|
| Xanthangummi | 0,20 |
| Magnesiumaluminiumsilicat | 0,40 |
| Aristoflex AVC | 0,70 |
| γ-Cyclodextrin (Fa. Wacker) | 2,00 |
| Glycerin | 2,00 |
| Panthenol | 1,50 |
| Chromoxid grün | 2,00 |
| Glitzerpigmente | 1,00 |
| Sicopearl Fantastico ® (BASF) | 2,50 |
| Parfüm, Konservierungsmittel, NaOH, Komplexbildner, | |
| Farbstoffe, Antioxidantien etc. | q.s. |
| Wasser | ad 100,00 |

### Beispiel 4:

| | Gew. % |
|---|---|
| Aristoflex AVC | 2,00 |
| Chitosan | 0,50 |
| Milchsäure (90%ig) | 0,30 |
| Glycerin | 3,00 |
| Perlglanzpigmente | 5,00 |
| Parfüm, Konservierungsmittel, NaOH, | |
| Farbstoffe, Antioxidantien, Pigmente etc. | q.s. |
| Wasser | ad 100,00 |

### Beispiel 5:

| | Gew. % |
|---|---|
| Ethanol | 2,00 |
| Aristoflex AVC | 1,00 |
| Hydroxyproylethylcellulose | 0,35 |
| Glycerin | 1,00 |
| Sorbit | 1,00 |
| Stärkeoctenylsulfat | 1,25 |
| Microna Matte Blue ® (Merck KgaA) | 5,00 |
| Talkum | 2,00 |
| Parfüm, Konservierungsmittel, Neutralisationsmittel, | |
| Farbstoffe, Antioxidantien, Pigmente etc. | q.s. |
| Wasser | ad 100,00 |

### Beispiel 6:

| | Gew. % |
|---|---|
| Perlglanzpigmente | 10,00 |
| BiOCI | 5,00 |
| PVP (Luviskol K 30) | 1,50 |
| Aristoflex AVC | 2,00 |
| Glycerin | 5,00 |
| Citronensäure | q.s. |
| Parfüm, Konservierungsmittel, Farbstoffe, | |
| Antioxidantien, etc. | q.s. |
| Wasser | ad 100,00 |

### Vergleichsbeispiel A (Eye Liner Gel) :

| | Gew. % |
|---|---|
| Perlglanzpigmente | 10,00 |
| Eisenoxide | 3,00 |
| Silica | 2,00 |
| Aristoflex AVC | 1,50 |
| Citronensäure | q.s. |
| Glycerin | 5,00 |
| PVP/VA Copolymer | 2,00 |
| Parfüm, Konservierungsmittel, Farbstoffe, NaOH, | |
| Komplexbildner, Antioxidantien, etc. | q.s. |
| Wasser | ad 100,00 |

### Beispiel 7 (Highlightening Gel):

| | Gew.% |
|---|---|
| Perlglanzpigmente | 6,00 |
| Aristoflex AVC | 2,70 |
| 1,3 Butylenglycol | 3,50 |
| Glycerin | 3,50 |
| Caprylsäure/Caprinsäuretriglyceride | 1,50 |
| Tocopherolacetat | 0,50 |
| Parfüm, Konservierungsmittel, NaOH, Komplexbildner, | |
| Farbstoffe, Antioxidantien, Pigmente etc. | q.s. |
| Wasser | ad 100,00 |

## Patentansprüche

1. Lidschattenzubereitung, **dadurch gekennzeichnet, dass** sie auf einem kosmetischen Gel basiert und einen Gehalt an einem oder mehreren Ammoniumacryloyldimethyltaurat/Vinylpyrrolidoncopolymeren, sowie einen Gehalt an einem oder mehreren Farbstoffen und/oder Farbpigmenten aufweist und die Gesamtmenge der Farbstoffe und farbgebenden Pigmente aus dem Bereich von 0,1 Gew.-% bis 30 Gew.-%, vorzugsweise von 0,5 bis 15 Gew.-%, insbesondere von 1,0 bis 10 Gew.-% gewählt wird, jeweils bezogen auf das Gesamtgewicht der Zubereitungen.

2. Lidschattenzubereitung nach Anspruch 1, **dadurch gekennzeichnet, dass** die Gesamtmenge des Gelbildners aus dem Bereich von 0,1 Gew.-% bis 10 Gew.-%, vorzugsweise von 0,2 bis 6 Gew.-%, insbesondere von 0,3 bis 5 Gew.-% gewählt wird, jeweils bezogen auf das Gesamtgewicht der Zubereitungen.

## Claims

1. An eye shadow preparation which is based on a cosmetic gel and contains one or more ammonium acryloyldimethyltaurate/vinylpyrrolidone copolymers, and has a content of one or more dyes and/or color pigments and wherein the total amount of dyes and color-imparting pigments is selected from the range from 0.1% by weight to 30% by weight, preferably from 0.5 to 15% by weight, in particular from 1.0 to 10% by weight, in each case based on the total weight of the preparations.

2. The eye shadow preparation as claimed in claim 1, wherein the total amount of the gel former is selected from the range from 0.1% by weight to 10% by weight, preferably from 0.2 to 6% by weight, in particular from 0.3 to 5% by weight, in each case based on the total weight of the preparations.

## Revendications

1. Préparation pour fard à paupières, **caractérisée en ce qu'**elle est à base de gel cosmétique et qu'elle présente une teneur en un ou plusieurs copolymères acryloyldiméthyltaurate d'ammonium/vinylpyrrolidone, ainsi qu'une certaine teneur en un ou plusieurs colorants et/ou pigments colorants, et **en ce que** la teneur totale en colorants et/ou pigments colorants est choisie dans la gamme de 0,1% à 30% en poids, de préférence de 0,5% à 15% en poids, en particulier de 1,0% à 10% en poids, à chaque fois par rapport au poids total des préparations.

2. Préparation pour fard à paupières selon la revendication 1, **caractérisée en ce que** la teneur totale en gélifiant est choisie dans la gamme de 0,1% à 10% en poids, de préférence de 0,2% à 6% en poids, en particulier de 0,3% à 5% en poids, à chaque fois par rapport au poids total des préparations.
